(19) Europäisches Patentamt European Patent Office Office européen des brevets

(11) **EP 3 838 273 B1**

(12) **EUROPEAN PATENT SPECIFICATION**

(45) Date of publication and mention of the grant of the patent:
**10.07.2024 Bulletin 2024/28**

(21) Application number: **19849696.0**

(22) Date of filing: **12.08.2019**

(51) International Patent Classification (IPC):
**A61K 31/4406** *(2006.01)* **A61K 31/47** *(2006.01)*
**A61P 35/00** *(2006.01)* **A61K 45/06** *(2006.01)*
**A61K 9/16** *(2006.01)* **A61K 9/20** *(2006.01)*
**A61K 9/28** *(2006.01)*

(52) Cooperative Patent Classification (CPC):
(C-Sets available)
**A61K 31/4406; A61K 9/1652; A61K 9/2054;**
**A61K 9/2886; A61K 9/2893; A61K 31/47;**
**A61K 45/06; A61P 35/00** (Cont.)

(86) International application number:
**PCT/CN2019/100175**

(87) International publication number:
**WO 2020/034916 (20.02.2020 Gazette 2020/08)**

(54) **COMBINATION OF CHIDAMIDE AND CHIAURANIB FOR USE IN THE TREATMENT OF HEPATOCELLULAR CARCINOMA**

KOMBINATION VON CHIDAMID UND CHIAURANIB ZUR VERWENDUNG BEI DER BEHANDLUNG VON HEPATOZELLULÄREM KARZINOM

COMBINAISON DE CHIDAMIDE ET DE CHIAURANIB POUR SON UTILISATION DANS LE TRAITEMENT D'UN CARCINOME HÉPATOCELLULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.08.2018 CN 201810943005**

(43) Date of publication of application:
**23.06.2021 Bulletin 2021/25**

(73) Proprietor: **Shenzhen Chipscreen Biosciences Co., Ltd.**
**Shenzhen, Guangdong 518057 (CN)**

(72) Inventors:
 • **LU, Xianping**
 **Shenzhen, Guangdong 518057 (CN)**
 • **NING, Zhiqiang**
 **Shenzhen, Guangdong 518057 (CN)**
 • **ZHOU, You**
 **Shenzhen, Guangdong 518057 (CN)**
 • **XIN, Lijun**
 **Shenzhen, Guangdong 518057 (CN)**
 • **WANG, Yanan**
 **Shenzhen, Guangdong 518057 (CN)**
 • **WANG, Shigang**
 **Shenzhen, Guangdong 518057 (CN)**
 • **PAN, Desi**
 **Shenzhen, Guangdong 518057 (CN)**
 • **SHAN, Song**
 **Shenzhen, Guangdong 518057 (CN)**

(74) Representative: **Lavoix**
**Bayerstraße 83**
**80335 München (DE)**

(56) References cited:
**WO-A1-2018/017858    WO-A2-2004/026234**
**CN-A- 101 837 129    CN-A- 102 441 167**
**CN-A- 103 432 077    CN-A- 104 771 363**
**US-A1- 2015 105 409**

**EP 3 838 273 B1**

- HE BOLIN ET AL: "Chidamide, a Novel Histone Deacetylase Inhibitor, Combined with Imatinib Induces Synthetic Lethality in Drug-Resistant Chronic Myeloid Leukemia", BLOOD, AMERICAN SOCIETY OF HEMATOLOGY, US, vol. 130, 8 December 2017 (2017-12-08), pages 4990, XP086633544, ISSN: 0006-4971, DOI: 10.1182/BLOOD.V130.SUPPL_1.4990.4990
- ANA-MARIA MARINO ET AL: "Enhanced effects by 4-phenylbutyrate in combination with RTK inhibitors on proliferation in brain tumor cell models", BIOCHEMICAL AND BIOPHYSICAL RESEARCH COMMUNICATIONS, vol. 411, no. 1, 25 January 2011 (2011-01-25), pages 208 - 212, XP028327125, ISSN: 0006-291X, [retrieved on 20110625], DOI: 10.1016/J.BBRC.2011.06.141
- YOU ZHOU ET AL: "CS2164, a novel multi-target inhibitor against tumor angiogenesis, mitosis and chronic inflammation with anti-tumor potency", CANCER SCIENCE, vol. 108, no. 3, 1 March 2017 (2017-03-01), JP, pages 469 - 477, XP055737667, ISSN: 1347-9032, DOI: 10.1111/cas.13141
- DENG MANMAN ET AL: "CS2164 exerts an antitumor effect against human Non-Hodgkin's lymphomasin vitroandin vivo", EXPERIMENTAL CELL RESEARCH, ELSEVIER, AMSTERDAM, NL, vol. 369, no. 2, 1 June 2018 (2018-06-01), pages 356 - 362, XP085432993, ISSN: 0014-4827, DOI: 10.1016/J.YEXCR.2018.05.038
- ANONYMOUS: "Sioroni in combination with cidabenamide for relapsed / refractory non-Hodgkin's lymphoma", 21 May 2019 (2019-05-21), pages 1 - 5, XP055686847, Retrieved from the Internet <URL:http://cancer123.com/treatment/1112>
- ANONYMOUS: "Chiauranib in Combination With Chidamide in Patients With Relapsed/Refra-ctory Non-Hodgkin's Lymphoma", pages 1 - 8, XP055686866, Retrieved from the Internet <URL:https://clinicaltrials.gov/ct2/show/NCT03974243>
- ANONYMOUS: "The role and prospects of selective histone deacetylase inhibitors in tumor immunotherapy", 12 December 2016 (2016-12-12), pages 1 - 4, XP055686869, Retrieved from the Internet <URL:http://www.chem17.com/tech_news/detail/1079799.html>
- NANJING KEBAI BIOTECHNOLOGY C: "The role and prospects of selective histone deacetylase inhibitors in tumor immunotherapy", CHEM17.COM, 12 December 2016 (2016-12-12), pages 1 - 4, XP055686869, Retrieved from the Internet <URL:http://www.chem17.com/tech_news/detail/1079799.html>

(52) Cooperative Patent Classification (CPC): (Cont.)

C-Sets
A61K 31/4406, A61K 2300/00;
A61K 31/47, A61K 2300/00

**Description**

**CROSS REFERENCE TO RELATED APPLICATIONS**

The application claims the priority to Chinese Patent Application No. 201810943005.6 titled "COMBINATION OF HISTONE DEACETYLASE INHIBITOR AND PROTEIN KINASE INHIBITOR AND PHARMACEUTICAL USE THEREOF", filed on Aug. 17, 2018 with the China National Intellectual Property Administration.

**FIELD**

The present disclosure relates to the technical field of small-molecule targeted anti-tumor medicine, and specifically relates to the use of a combination of a histone deacetylase inhibitor and a kinase inhibitor in the preparation of a medication for treating or preventing a tumor, and a pharmaceutical composition containing a histone deacetylase inhibitor and a kinase inhibitor as active pharmaceutical ingredients. The present invention particularly relates to a pharmaceutical composition containing Chiauranib or a pharmaceutically acceptable salt thereof and Chidamide or a pharmaceutically acceptable salt thereof as active ingredients, for use in a method of treating or preventing hepatocellular carcinoma.

**BACKGROUND**

Cancer is a major disease threating human health. The treatment of cancer has attracted the attention of the whole world. Conventional chemotherapeutic drugs non-specifically block cell division to cause cell death, but they also damage normal human cells while killing tumor cells. In addition, many cytotoxic drugs have a limited treatment range, and are prone to cause adverse reactions, which can increase drug resistance after long-term administration.

Various types of anti-tumor drugs have been developed in the prior art, among which histone deacetylase (HDAC) inhibitors are an important class of anti-tumor compounds.

Histone deacetylase (HDAC) is a class of protease that plays an important role in the structural modification of chromosomes and the regulation of gene expression. In general, the histone acetylation facilitates the dissociation of DNA and histone octamers and nucleosome structure relaxation, so that various transcription factors and cooperative transcription factors can specifically bind to DNA binding sites and activate gene transcription. In the nucleus, histone acetylation and deacetylation are in dynamic equilibrium, regulated by histone acetyltransferases (HATs) and HDACs. HATs transfer the acetyl group from Acetyl-CoA to the specific lysine residues at the amino terminus of histones. HDACs make histones deacetylation and bind tightly to negatively charged DNA, thereby chromatin is more compact and curved, resulting in transcriptional repression. In cancer cells, the overexpression of HDAC leads to the increase of deacetylation. By restoring the positive charge of histones, the interaction between DNA and histones is increased, and the loose nucleosomes become compact, which is unfavorable for the expression of specific genes, including some tumor suppressor genes. HDAC inhibitors, as a new class of anti-tumor drugs, can increase the histone acetylation in specific regions of chromatin, thereby regulating the expression and stability of proteins related to cell apoptosis and differentiation, and inducing cell apoptosis and differentiation. HDAC inhibitors not only have a good therapeutic effect on a variety of hematological tumors and solid tumors, but also have the advantages of relatively high selectivity to tumor cells and low toxicity.

Literature has shown that inhibiting the activity of HDAC can effectively inhibit the growth, metastasis and invasion of tumor cells. HDAC inhibitors have become important anti-tumor candidates. Studies have shown that HDAC inhibitors can effectively inhibit the proliferation of tumor cells, induce tumor cell apoptosis and differentiation as well as anti-angiogenesis, and have inhibitory effects on tumor cell migration, invasion and metastasis.

HDAC inhibitors activate tumor suppressor genes by regulating the acetylation and deacetylation of lysine residues at the N-terminal of histones, thereby inhibiting tumor cell growth and inducing tumor cell apoptosis (Apoptosis on hepatoma cells but not on primary hepatocytes by histone deacetylase inhibitors valproate and ITF2357. J Hepatol, 2005, 42: 210-217). Yindong Kang et al., in 2009, published a research review on the immunomodulation effect of histone deacetylase inhibitors through dendritic cells. (Yindong Kang et al., Studies on the Immunomodulation Effect of Histone Deacetylase Inhibitors through Dendritic Cells, Chinese Journal of Transplantation, Volume 3, Issue 2, 2009). This review described the relationship between histone deacetylase inhibitors and dendritic cells (DCs) as well as immune regulation. HDAC inhibitors exhibit strong anti-tumor activity at high concentrations, and a variety of immunomodulatory effects at low concentrations. When HDAC inhibitors directly act on DCs, they weaken the ability of DCs to migrate to chemokine CCL19 during maturation, thereby restricting DCs from reaching and entering into lymphoid organs to exert their antigen-presenting functions. HDAC inhibitors affect the differentiation, maturation and migration of DCs, and interfere with the antigen presentation process of DCs, as the most important antigen-presenting cell, which play an important role on the activation of antigen-specific T lymphocytes. In general, HDAC inhibitors directly act on DCs to suppress the immune response.

In recent years, studies on the functions of HDAC have progressed rapidly. It gradually becomes a research hotspot in this field to develop a selective HDAC inhibitor. Currently, a variety of HDAC inhibitors have been already commercially available both at home and abroad, e.g., vorinostat, chidamide. However, the existing HDAC inhibitors still need to be improved in the aspects of activity, efficacy, drug metabolism and side effects. An anti-tumor drug with stronger efficacy and better effect is urgently required in the prior art, to meet people's increasing health needs.

Zhou Y., et al. (Cancer Science, 2017, 108(3), pages 469-477) and Manman D., et al. (Experimental Cell Research, 2018, 369(2), pages 356-362) disclose that the novel Aurora B protein kinase inhibitor CS2164 (chiauranib) has antitumour effects both *in vivo* and *in vitro*. They report for example that this compound inhibits tumour growth of hepatocellular cell lines, and exhibits potent activity against tumor angiogenesis and mitosis in hepatoma.

## SUMMARY

The present invention is defined by the claims. Any subject-matter falling outside the scope of the claims is provided for information only. Methods of treatment of the human body by therapy, referred to in this description, are not part of the present invention as such, but are described here in relation to compounds and pharmaceutical compositions for use in said methods of treatment of the human body by therapy, according to the present invention. The inventors unexpectedly found in the study that the combination of a histone deacetylase inhibitor and a protein kinase inhibitor can achieve synergistic anti-tumor effects.

The present invention relates to a combination of a histone deacetylase inhibitor and a protein kinase inhibitor for use in treating or preventing hepatocellular carcinoma; wherein the histone deacetylase inhibitor is Chidamide or a pharmaceutically acceptable salt thereof; and the protein kinase inhibitor is Chiauranib or a pharmaceutically acceptable salt thereof.

Protein kinases are a family of enzymes that catalyze the phosphorylation of specific residues in proteins. They are broadly classified into tyrosine and serine/threonine-protein kinases, which represent a big family of proteins that play an important role in regulating various cellular processes and maintaining cell functions. Protein kinases are enzymes associated with signal transduction pathways, which catalyze the transfer of the terminal phosphate of ATP to the hydroxyl group of tyrosine, serine and/or threonine residues of the proteins. Overexpression or improper expression of normal or mutant protein kinases in mammals has been extensively studied, and has been shown to play an important role in the development of many diseases including cancer. These kinases partially include, but not limited to non-receptor tyrosine kinases, e.g., Janus kinase family (Jak1, Jak2, Jak3 and Tyk2); receptor tyrosine kinases, e.g., platelet-derived growth factor receptor kinases (PDGFR); and serine/threonine-protein kinases, e.g., b-RAF. Abnormal kinase activity is observed in many diseases, including benign and malignant proliferative disorders and diseases caused by inappropriate activation of the immune and nervous systems.

As a large family of structurally related enzymes, protein kinases are responsible for controlling a variety of signal transduction processes in cells. (See, for example, Hardie and Hanks, The Protein Kinase Facts Book, I and II, Academic Press, San Diego, Calif., 1995). Protein kinases are thought to have evolved from a common ancestral gene due to the conservation of their structure and catalytic function. Almost all kinases contain a similar catalytic domain with 250-300 amino acids. Kinases can be categorized into different families by their phosphorylation receptors (e.g. protein-tyrosine, protein-serine/threonine, lipids, etc.). Sequence motifs have been identified that generally correspond to each of these families. (see, for example, Hanks and Hunter, (1995), FASEB J.9:576-596; Knighton etc., Science, (1991) 253:407-414; Hiles etc., Cell, (1992), 70:419-429; Kunz etc., Cell (1993), 73:585-596; Garcia-Bustos etc., EMBO J., (1994), 13:2352-2361).

Many diseases are associated with inappropriate kinase activity caused by mutation, overexpression or inappropriate regulation, abnormal regulation or dysregulation, as well as overproduction or underproduction of growth factors or cytokines, including but not limited to cancer and other diseases. Protein kinases have become a class of important enzymes as targets for therapeutic intervention. Particularly, tyrosine kinase cKit over-activation is associated with hematological malignancies and is a target for cancer therapy (Heinrich, Griffith etc., Blood 2000, 96 (3): 925-32). Similarly, JAK3 signaling is involved in leukemia and lymphoma, which is currently used as a potential therapeutic target (Heinrich, Griffith etc., 2000). Protein kinases also play a key role in cell-cycle regulation. It has been found that a variety of diseases, including many types of cancers, may be caused by the defects in various components of signal transduction pathways (Gaestel et al., Current Medicinal Chemistry, (2007) 14: 2214-2234). In recent years, the protein kinases related to oncogenic signal transduction pathways have become important drug targets in the treatment of various diseases including many types of cancers. A variety of protein kinase inhibitors also have been used as anti-tumor drugs.

According to the present invention, the histone deacetylase inhibitor is Chidamide or a pharmaceutically acceptable salt thereof, and the kinase inhibitor is Chiauranib or a pharmaceutically acceptable salt thereof. More preferably, the histone deacetylase inhibitor is Chidamide, and the kinase inhibitor is Chiauranib.

In another aspect, the present disclosure provides a pharmaceutical composition, comprising a histone deacetylase inhibitor and a protein kinase inhibitor as active pharmaceutical ingredients, and optionally comprising pharmaceutically acceptable excipients and/or carriers, wherein the protein kinase inhibitor is Chiauranib or a pharmaceutically acceptable salt thereof, and the histone deacetylase inhibitor is Chidamide or a pharmaceutically acceptable salt thereof. In a particular embodiment, the active pharmaceutical ingredients consist of Chiauranib and Chidamide.

In a particularly preferred embodiment of the present disclosure, the pharmaceutical composition is provided in dosage unit form, comprising 5-100 mg of Chidamide and 5-100 mg of Chiauranib. More preferably, the amount of Chidamide and Chiauranib is 5-60 mg and 10-100 mg, respectively.

In some embodiments of the present disclosure, in the pharmaceutical composition, the pharmaceutically acceptable excipients and/or carriers thereof comprise povidone, copovidone, hydroxypropylmethyl celluloses and polyvinyl capralactam-polyvinyl acetate-polyethylene glycol graft copolymers (e.g. Soluplus®). In some other embodiments, the pharmaceutically acceptable excipients and/or carriers comprise microcrystalline cellulose, povidone, copovidone, lactose, mannitol, crospovidone and sodium carboxymethyl cellulose.

The pharmaceutical composition of the present disclosure is preferably in a form of granules, solid dispersions, capsules or tablets.

In another aspect, the present disclosure provides a kit for use in treating or preventing hepatocellular carcinoma, comprising a histone deacetylase inhibitor and a protein kinase inhibitor as active ingredients, as defined in the appended claims, wherein the histone deacetylase inhibitor and the protein kinase inhibitor can be administered simultaneously or sequentially.

The kit of the present disclosure comprises Chidamide or a pharmaceutically acceptable salt thereof and Chiauranib or a pharmaceutically acceptable salt thereof as the active ingredients, which can be administered simultaneously or sequentially. In a particularly preferred aspect, the kit of the present disclosure comprises Chidamide or a pharmaceutically acceptable salt thereof administered first and Chiauranib or a pharmaceutically acceptable salt thereof administered second as the active ingredients.

The inventors unexpectedly found that the combination of a histone deacetylase inhibitor and a protein kinase inhibitor according to the appended claims, has significant synergistic anti-tumor effects which has been confirmed in the nude mouse experiments, shown that the combined administering of the two ingredients can synergistically induce the apoptosis of cancer cells and synergistically inhibit the colony formation of tumor cells. The inventors also unexpectedly found that pre-treatment with the histone deacetylase inhibitor chiauranib can increase the sensitivity of the cells to the protein kinase inhibitor chidamide, and more effectively enhance the anti-tumor effects of the protein kinase inhibitor.

It was found in the MTS, colony formation, cell cycle analysis in flow cytometry, Caspase 3/7 and other experiments of the present disclosure that the combined administration of a histone deacetylase inhibitor and a protein kinase inhibitor according to the appended claims can synergistically induce the cell cycle inhibition and apoptosis of the hepatocellular carcinoma cell lines, e.g. Bel-7404 and Bel-7402. Such synergistic anti-tumor efficacy by the combination of a histone deacetylase inhibitor and a protein kinase inhibitor has also been proved by the Bel-7404 cell xenograft model in nude mice.

## BRIEF DESCRIPTION OF DRAWINGS

FIG 1 shows the anti-tumor efficacy of a protein kinase inhibitor (Chiauranib) synergistically sensitized by a histone deacetylase inhibitor (Chidamide);

FIG 2 shows that the combination of a histone deacetylase inhibitor and a protein kinase inhibitor can synergistically inhibit tumor cell clonogenicity as shown in crystal violet assay;

FIG 3 shows that the combination of a histone deacetylase inhibitor and a protein kinase inhibitor can enhance the inhibition of the tumor cell cycle tested by flow cytometry assay with propidium iodide (PI) ;

FIG 4 shows that the combination of a histone deacetylase inhibitor and a protein kinase inhibitor can enhance the induction of tumor apoptosis tested by the Caspase 3/7 assay;

FIG 5 shows that the pretreatment with a histone deacetylase inhibitor (Chidamide) can enhance the anti-tumor effect of a protein kinase inhibitor (Chiauranib) in the sequential administration experiments;

FIG 6 shows the synergistic anti-tumor efficacy of the combination of a histone deacetylase inhibitor (Chidamide) and a protein kinase inhibitor (Chiauranib) in the nude mouse models.

## DETAILED DESCRIPTION

The terms used herein are used for describing specific embodiments, but is not intended to limit the present disclosure. As used herein, the singular forms "a" and "the" are also intended to include plural forms, unless there is a clear indication in the context. In addition, the terms "include", "comprise", "contain" or variations thereof in the specification and/or claims of the present disclosure are intended to be used in a similar manner as the term "comprise".

As used herein, the terms "treat", "alleviate" and "improve" can be used interchangeably. These terms refer to the methods related to beneficial or expected effects (including but not limited to therapeutic benefit and/or preventive benefit).

As used herein, the term "anti-tumor" refers to the treatment, alleviation or improvement of "tumor condition". The term "tumor condition" refers to the existence of cells with abnormal growth characteristics such as uncontrolled proliferation, infinite proliferation, metastatic potential, rapid growth and high proliferation rate, disordered oncogenic signaling and certain unique morphology. It includes but not limited to the growth of the following cells: (1) benign or malignant cells (e.g. tumor cells) associated with overexpression of histone deacetylases, tyrosine or serine/threonine-protein kinases; (2) benign or malignant cells (e.g. tumor cells) associated with the abnormally high activity of histone deacetylases, tyrosine or serine/threonine-protein kinases.

Those skilled in the art can understand that, in the drug combination or pharmaceutical composition of the present disclosure, the active pharmaceutical ingredients are used at an effective amount or a therapeutically effective amount. The terms "effective amount" or "therapeutically effective amount" refers to the amount of the inhibitor described herein that is sufficient to achieve the intended result (including but not limited to the application in the treatment of diseases) as defined below. The therapeutically effective amount may vary according to the intended application (*in vitro* or *in vivo*), or the subject being treated and the disease condition, such as the body weight and age of the subjects, the disease severity, the route of administration, etc, which can be easily determined by a skilled person in the art. The term also applies to the dosage that can induce a specific response (e.g., reduction of proliferation or down-regulation of target protein activity) in the target cell. The specific dosage may vary depending on the particular compound selected, the dosing regimen to be followed, whether it is administered in combination with other compounds, the timing of administration, the tissue to be administered, and the physical delivery system for carrying it.

"Synergy" or "synergistic effect" means that when one active pharmaceutical ingredient combined with an effective amount of another active pharmaceutical ingredient or another therapy, it produces a better effect than using each of them alone. In some embodiments, the synergistic use of active pharmaceutical ingredients in effective therapeutic amounts or therapies produces a better effect than the additive effect of each of the two active pharmaceutical ingredients or therapies when used alone.

The term "inhibitor" refers to a compound capable of inhibiting the biological function of a target protein by inhibiting the activity or expression of the target protein. The biological activity of the inhibitor is related to the development, growth or spread of a tumor or undesired immune responses in an autoimmune disease.

The terms "combined administration", "co-administration" and their grammatical equivalents involve applying two or more pharmaceutically active ingredients to a subject so that the pharmaceutically active ingredients and/or the metabolites thereof can be simultaneously present in the animal. The combined administration includes administering the active ingredients concurrently or at different times as separated compositions, or administering a composition comprising the two active ingredients thereof. The co-administrated active pharmaceutical ingredients can be in the same preparation, or in different preparations, or in the same product, for example, as a form of a kit.

As used herein, "therapeutic effect" includes the therapeutic and/or preventive benefits as described above. The preventive effect includes delaying or eliminating in appearance of the disease or condition, delaying or eliminating the onset of the disease or condition, slowing down, stopping or reversing the progression of the disease or condition, or any combination thereof.

The term "pharmaceutically acceptable salt" refers to salts derived from a variety of organic and inorganic counterions well known in the art. The pharmaceutically acceptable salts include pharmaceutically acceptable acid addition salts and base addition salts. The acid addition salts can be formed with inorganic and organic acids. The inorganic acids capable of deriving salts therefrom include, for example, hydrochloric acid, hydrobromic acid, sulfuric acid, nitric acid, phosphoric acid, and the like. The organic acids capable of deriving salts therefrom include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. The pharmaceutically acceptable base addition salts may be formed with inorganic and organic bases. The inorganic bases capable of deriving salts therefrom include, for example, sodium, potassium, lithium, ammonium, calcium, magnesium, iron, zinc, copper, manganese, aluminum, and the like. The organic bases capable of deriving salts therefrom include, for example, primary amines, secondary amines and tertiary amines, substituted amines including natural substituted amines, cyclic amines, basic ion exchange resins, and the like, especially such as isopropyl amine, trimethylamine, diethylamine, triethylamine, tripropylamine and ethanolamine. In some embodiments, the pharmaceutically acceptable base addition salts selected from ammonium, potassium, sodium, calcium and magnesium salts.

The "pharmaceutically acceptable excipient and/or carrier" include any and all solvents, dispersion media, coatings, antibacterial agents and antifungal agents, isotonic regulators and absorption delaying agents, and the like. These media and agents used in pharmaceutically active substances are well known in the art. Any conventional media or agents can be used in the therapeutic compositions of the present disclosure, except for those incompatible with the active ingredients. Supplementary active ingredients can also be incorporated into the compositions thereof.

In the present disclosure, the tumors include the following diseases or conditions: breast cancer; ovarian cancer; uterine cancer; cervical cancer; prostate cancer; bladder cancer; leukocythemia including acute myeloid leukemia (AML), acute lymphocytic leukemia, chronic lymphocytic leukemia, chronic myeloid leukemia, hairy cell leukemia, osteomyelodysplasia, myeloproliferative diseases, acute myeloid leukemia (AML), chronic myelogenous leukemia (CML), mastocytosis, chronic lymphocytic leukemia (CLL), multiple myeloma (MM) and myelodysplastic syndromes (MDS); bone cancer; lung cancer; skin cancer including basal cell carcinoma, melanoma, and squamous-cell carcinoma; retinoblastoma; skin or intraocular (ocular) melanoma; primary hepatic carcinoma; renal carcinoma; thyroid carcinoma; AIDS-related lymphadenoma, such as diffuse large B-cell lymphoma, immunoblastic B-cell lymphoma and small noncleaved cell lyrephoma; kaposissarcoma; central nervous system cancer, such as primary brain tumor including neuroglioma; peripheral nervous system cancer, including neurofibromatosis and neurilemoma, malignant fibrous cell tumor, malignant fibrous histiocytoma, malignant meningioma, malignant mesothelioma and malignant mixed mullerian tumor; oral and oropharyngeal cancer; gastric cancer; testicular cancer; thymic carcinoma; rectal cancer and colon cancer. The cancer treated by the present invention is hepatocellular carcinoma.

The combination treatment according to the present disclosure is effective in a wide dosage range. For example, when used to treat adults, the daily dose of the histone deacetylase inhibitor and protein kinase inhibitor can be 1 to 100 mg, preferably 5 to 100 mg, respectively. In a particular embodiment, a combination of Chidamide and Chiauranib is used, wherein the daily dosage of Chidamide is 5 to 60 mg, and the daily dose of Chiauranib is 10 to 100 mg, respectively. The exact dose can be easily determined by those skilled in the art according to the selected active pharmaceutical ingredient, the route of administration, the form of the compound to be administrated, the subject to be treated, the weight of the subject to be treated, and the preference and experience of the doctor.

The pharmaceutical combination of the present disclosure can also be used together with other active pharmaceutical ingredients with anti-tumor activity. Accordingly, the pharmaceutical composition or kit of the present disclosure can further contain other active pharmaceutical ingredient(s) with anti-tumor activity.

In some embodiments, the pharmaceutical composition can be suitable for oral administration, for example, in the form of granules, capsules, or tablets. The pharmaceutical composition of the present disclosure suitable for oral administration can be in dispersed dosage forms, such as capsules or tablets, liquids or sprays, each containing a predetermined amount of active ingredients, as powders or in granules, solutions, or suspensions in aqueous or non-aqueous liquids, oil-in-water emulsions or water-in-oil liquid emulsions, including liquid dosage forms (such as suspensions or slurries) and oral solid dosage forms (such as tablets or bulk powders). The term "tablet" as used herein generally refers to tablets, caplets, capsules (including soft gelatin capsules), and lozenges. The oral dosage forms can be provided as tablets, pills, dragees, capsules, emulsions, lipophilic and hydrophilic suspensions, liquids, gels, syrups, slurries, suspensions, etctaken orally by an individual or patient to be treated. Such dosage forms can be prepared by any pharmaceutical method. Suitable excipients can be fillers, such as sugars, including lactose, sucrose, mannitol or sorbitol; cellulose preparations, such as corn starch, wheat starch, rice starch, potato starch, gelatin, tragacanth, methylcellulose, hydroxypropyl methyl cellulose, sodium carboxymethyl cellulose and/or polyvinylpyrrolidone (PVP). Generally speaking, the composition is prepared by uniformly and tightly mixing the active ingredient with liquid carriers or finely crushed solid carriers or both, and then shaping the product into the desired form if necessary. For example, it can be prepared into a tablet by compressing or molding optionally with one or more accessory ingredients. It also can be prepared into a tablet by compressing the active ingredient in a free-flowing form such as powder or granules using a suitable machine. The active ingredient thereof is optionally mixed with excipients such as, but not limited to, binders, lubricants, inert diluents and/or surfactants or dispersants. Moulded tablets can be prepared by moulding a mixture of the powdered compound moistened with an inert liquid diluent in a suitable machine. The carrier can take various forms according to the preparation form required for administration. When the composition is prepared for oral dosage forms, any conventional pharmaceutical medium can be used as a carrier. In the case of oral liquid preparations (such as suspensions, solutions and elixirs) or aerosols, such as water, glycols, oils, alcohols, flavoring agents, preservatives, coloring agents, etc can be used as carriers. In the case of oral solid preparations, in some embodiments where lactose is not used, for example, starches, sugars, microcrystalline cellulose, diluents, granulating agents, lubricants, binders, and disintegrating agents can be used as carriers. For example, suitable carriers for solid oral preparations include powders, capsules and tablets. If needed, the tablets can be coated by a standard aqueous or non-aqueous technique.

The composition can further comprise one or more pharmaceutically acceptable additives and excipients. Such additives and excipients include, but are not limited to, adhesion preventives, anti-foaming agents, buffers, polymers, antioxidants, preservatives, chelating agents, viscosity modifiers, tonicifiers, flavoring agents, coloring agents, flavor enhancers, opacifiers, suspending agents, binders, fillers, plasticizers, lubricants and mixtures thereof.

Experiments

Experimental materials

Human hepatoma cell line Bel-7402 and Bel-7404 were purchased from the Cell Resource Center of Shanghai Institutes for Biological Sciences, CAS, and routinely cultured at 37°C under 5%$CO_2$, in a culture medium of RPMI-1640 (Gibco) containing 10% fetal bovine serum (FBS; Gibco) and 1% Penicillin-Streptomycin (HyClone). Trypsin was purchased from Gibco. Crystal violet, RNase A (10 mg/mL) solution, propidium iodide (PI), Triton X-100 were purchased from Sangon Biotech (Shanghai) Co., Ltd.. MTS cell viability assay kits, Caspase-Glo 3/7 assay kits were purchased from Promega. Nude mice were purchased from Guangdong Medical Laboratory Animal Center.

Example 1. MTS assays

The experimental method

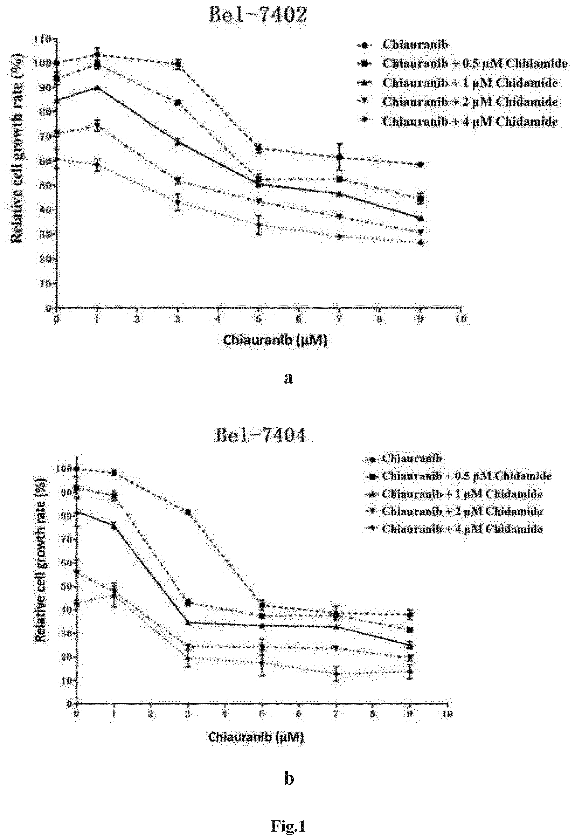

The Bel-7402, Bel-7404 cell lines were digested by trypsin, collected and counted, and then seeded in a 96-well cell culture plate at the density of 3,000 cells/180 $\mu$L in each well, and cultured at 37°C under 5% $CO_2$. After cultured overnight, the cells were administered according to the grouping and the final concentrations shown in FIG 1 (the final volume of each well after administration was 200 $\mu$L). The dose for each group was arranged 3 replicate wells. After the cells were treated with the drugs for 120 hours, the culture media in the 96-well plate were discarded, and 100 $\mu$L MTS reagents for detecting cell viability assay containing 89.5 $\mu$L phenol red-free RPMI-1640, 10 $\mu$L MTS and 0.5 $\mu$L PMS were added to each well, while the same volume of MTS reagents for detecting the cell viability assay was added to the wells without seeding cells as detection background ($OD_{490-BLK}$). After incubation at 37°C for about 1 hour, the absorbance of each well was read at 490 nm wavelength with a microplate reader. The $OD_{490-T}$ value of each administered well and the $OD_{490-T0}$ value of the negative control well after background deduction were obtained by subtracting $OD_{490-BLK}$ from the absorbance reading value of each well.

The relative survival rate of the cells in each administered well was calculated according to the following formula:

$$\text{Survival rate} = OD_{490\text{-}T} / OD_{490\text{-}T0} \times 100\%$$

The experimental result

As shown in FIG 1, in Bel-7402 and Bel-7404 cell lines, both monotreatment of Chiauranib and monotreatment of Chidamide showed a certain inhibitory effect on tumor cell proliferation, which were dose-dependent. It should be noted that in Bel-7402, when the effect dose of Chiauranib reached 3 $\mu$M and the effect dose of Chidamide reached 0.5, 1 or 2 $\mu$M, respectively, the two drugs exhibited a significant synergistic effect, that is, at the same dose, the efficacy of the combination of the two drugs was better than the sum of the efficacy of the two single drugs used separately. In Bel-7404, when the effect dose of Chiauranib reached 3 $\mu$M and the effect dose of Chidamide reached 0.5 or 1 $\mu$M, the two drugs exhibited a more significant synergistic effect than that in Bel-7402.

Example 2. Colony forming assays

The experimental method

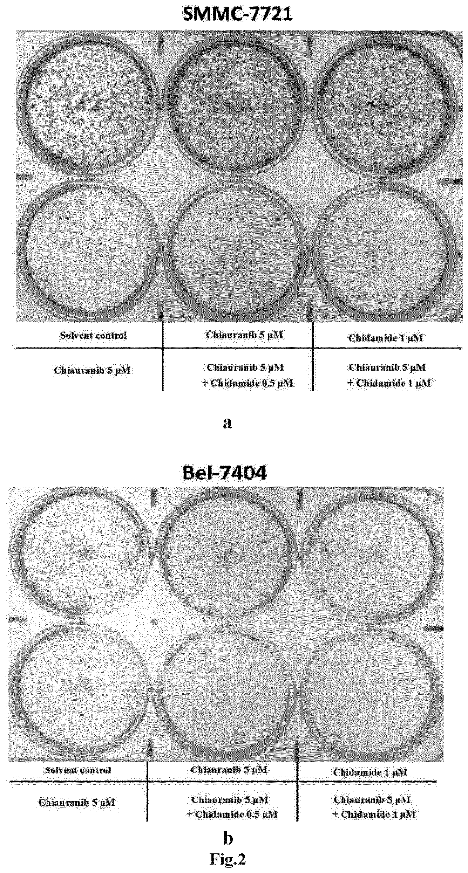

The SMMC-7721, Bel-7404 cell lines at the log phase stage were digested by trypsin, collected and counted, then seeded in a 6-well cell culture plate at the density of 500 cells/1.8 mL in each well, and cultured at 37°C under 5% $CO_2$. After cultured overnight, the cells were administered according to the grouping and the final concentration dosages shown in FIG 2 (the final volume of each well after administration was 2 mL). The culture media and drugs were replaced every 3 days to keep the culture system and the dosage for each well stable.

After cultured for 2-3 weeks, when there were visible cell colonies in the culture plate, the culture was terminated. The supernatants were discarded, and the plate was washed carefully with PBS twice. 1 mL of 75% ethanol solution was added to each well to fix the cells, and the fixative solution was discarded after 15 min. Then the cells were stained for 15 min by adding 1 mL of crystal violet solution to each well, and the staining solution was washed slowly with running water and air dried. The stained positive cell colonies in each well of the culture plate were photographed to compare their density.

The experimental result

As shown in FIG 2, in SMMC-7721, when Chidamide monotreated on cells at a dose of 0.5 or 1 $\mu$M, it had no significant effect on the formation of cell colonies. The density of positive colonies stained with crystal violet in the two wells was equivalent to that in the control well where an equal volume of DMSO solvent was added to. However, 5 $\mu$M dose of Chiauranib monotreatment had a relatively significant effect on the formation of cell colonies. When 0.5 or 1 $\mu$M dose of Chidamide was added with Chiauranib at such dose, the formation of cell colonies was inhibited more significantly, and the inhibition was positively correlated with the dose of Chidamide. In Bel-7404, when Chidamide monotreated on cells at a dosage of 0.5 or 1 $\mu$M, or Chiauranib monotreated on cells at a dosage of 3 $\mu$M, they all affected somewhat on the formation of cell colonies, while the cell colony formation was inhibited more significantly as the two drugs combined on the cells. Thus, the combination of Chidamide and Chiauranib had a synergistic sensitive effect on inhibition of the colonies of SMMC-7721 and Bel-7404.

Example 3. The cell cycle analyzed by the flow cytometry assay with PI staining

The experimental method

The cells of Bel-7404 at the log phase stage were digested by trypsin, collected and counted. The cells were seeded in a 6-well plate at the density of $10^6$ cells per well in a total of 18 wells. After normally cultured overnight, the seeded cells were divided into 6 groups, namely, solvent control group, 3 $\mu$M Chiauranib group, 0.5 $\mu$M Chidamide group, 1 $\mu$M Chidamide group, the combination of 3 $\mu$M Chiauranib and 0.5 $\mu$M Chidamide group, and the combination of 3 $\mu$M Chiauranib and 1 $\mu$M Chidamide group. Each group was arranged 3 repeating wells, and the corresponding compounds were added according to the above final concentrations. After cultured for 48 hours, the cell samples were collected by trypsinization and centrifuged at 800 rpm for 10 min. Each sample was fully resuspended in 300 $\mu$L phosphate buffered saline (PBS), dropped into 700 $\mu$L pre-cooled absolute ethanol dropwise, and then mixed uniformly by gentle inversion several times, to disperse and fix the cells. The fixed samples were allowed to stand at 4°C for more than 12 hours, and analyzed by the flow cytometry assay within 1 week.

PBS, 10 mg/mL PI stock solution, 10 mg/mL RNase A solution and Triton X-100 were mixed at a ratio of 1000 : 5 : 2 : 1 to form a working dye solution. The fixed cell samples were centrifuged at 4°C, 1000 rpm for 10 min, and the supernatants were completely discarded and washed twice with PBS. Each sample was resuspended in 300 $\mu$L of the above working dye solution. After incubated at 37°C for 30 minutes in the dark, the cells were filtered with a 200-mesh stainless steel screen. The filtrate was analyzed for cell cycles by the flow cytometry assay (each sample counted 20,000 cells).

The experiment result

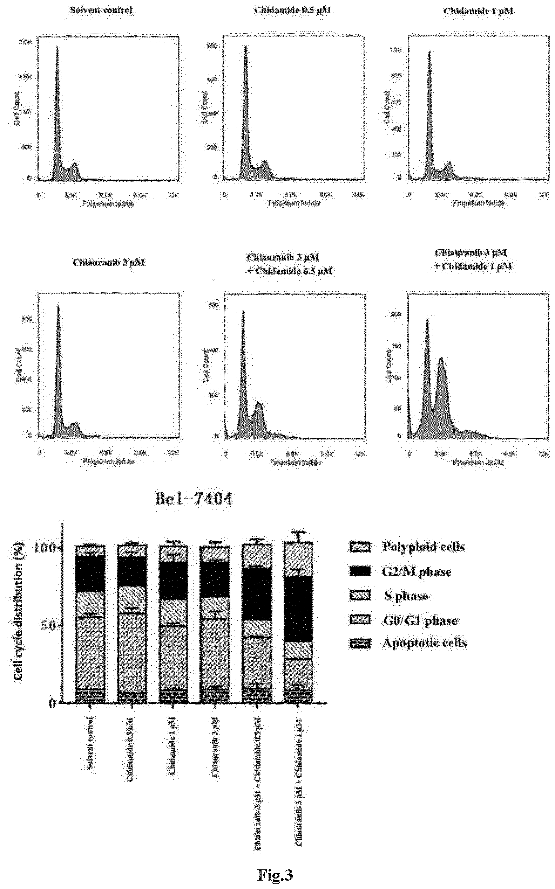

As shown in FIG 3, in Bel-7404, compared with the solvent control group, the cell cycle of the 0.5 $\mu$M Chidamide group was almost not affected. The cells in G2/M phase and polyploid cells slightly increased in the 1 $\mu$M Chidamide group and the 3 $\mu$M Chiauranib group. When 3 $\mu$M Chiauranib was combined with 0.5 $\mu$M or 1 $\mu$M Chidamide, the cells had a more significant G2/M phase arrest and an increase in the proportion of polyploid cells, indicating that the combination of Chidamide and Chiauranib had an anti-tumor effect of synergistically enhancing the cell cycle inhibition and synergistically promoting cell polyploidization.

Example 4. The cell apoptosis analyzed by Caspase 3/7 assay

The experimental method

The cells of Bel-7404 at the log phase stage were digested by trypsin, collected and counted. The cells were seeded in a 96-well plate at density of 2000 cells per well in a total of 18 wells. After normally cultured overnight, the seeded cells were divided into 6 groups, namely, the blank group, the solvent control group, the 3 $\mu$M Chiauranib group, the 0.5 $\mu$M Chidamide group, the combination of 3 $\mu$M Chiauranib and 0.5 $\mu$M Chidamide group and the positive control group. Each group was arranged 3 repeating wells, and the corresponding compounds were added according to the above final concentrations and co-cultured for 48 hours.

Caspase-Glo3/7 buffer and Caspase-Glo3/7 lyophilized powder as a substrate were equilibrated to 18-22°C. Then, the Caspase-Glo3/7 buffer was poured into a brown bottle containing the Caspase-Glo3/7 substrate, and mixed them by vortexingg or inverting until the substrate was thoroughly dissolved to form the Caspase-Glo reagent.

The 96-well plates containing the treated cells were taken out of the incubator and equilibrated to room temperature. 70 $\mu$L Caspase-Glo reagent (culture media : Caspase-Glo reagent = 1 : 1) was added to each well of the 96-well plate containing 70 $\mu$L of the blank control, the solvent control group, the drug treated groups and the positive control group. Contents of each well were mixed gently using a plate shaker at 300-500 rpm for 30 s. The cells were incubated at room temperature (18-22°C) for 30 min to 3 h. The fluorescence value of each sample was measured by a luminometer. The fluorescence value can directly reflect the proportion of apoptotic cells in each well, and can be used to calculate and compare the cell viability of the different groups.

The experimental result

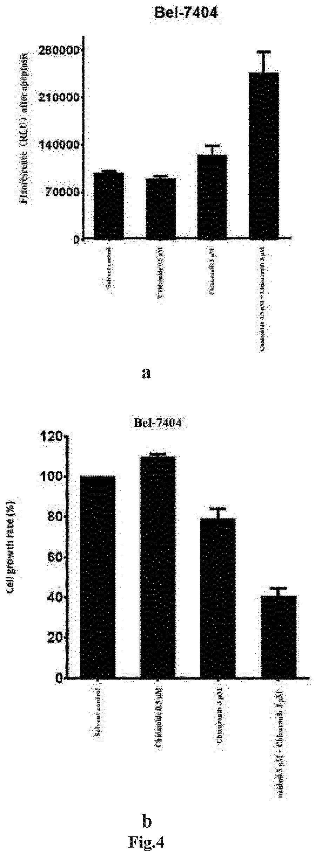

As shown in FIG 4, in Bel-7404, after the cells were treated with 0.5 $\mu$M Chidamide alone, the proportion of apoptotic cells had no significant difference from the solvent control group with the equal volume of DMSO. The monotreatment with 3 $\mu$M Chiauranib increased the proportion of apoptotic cells to a certain extent. When the two drugs at the above doses were combined to treat the cells, the proportion of apoptotic cells was significantly increased, which was significantly higher than the sum of the proportion of apoptosis increased by the two single drugs separately. The two drugs synergistically promoted tumor cell apoptosis. Accordingly, the combination caused an inhibition of tumor cell activity that was significantly greater than the sum of inhibition caused by using each of the two drugs alone.

Example 5. Sequential administeration experiment

The experimental method

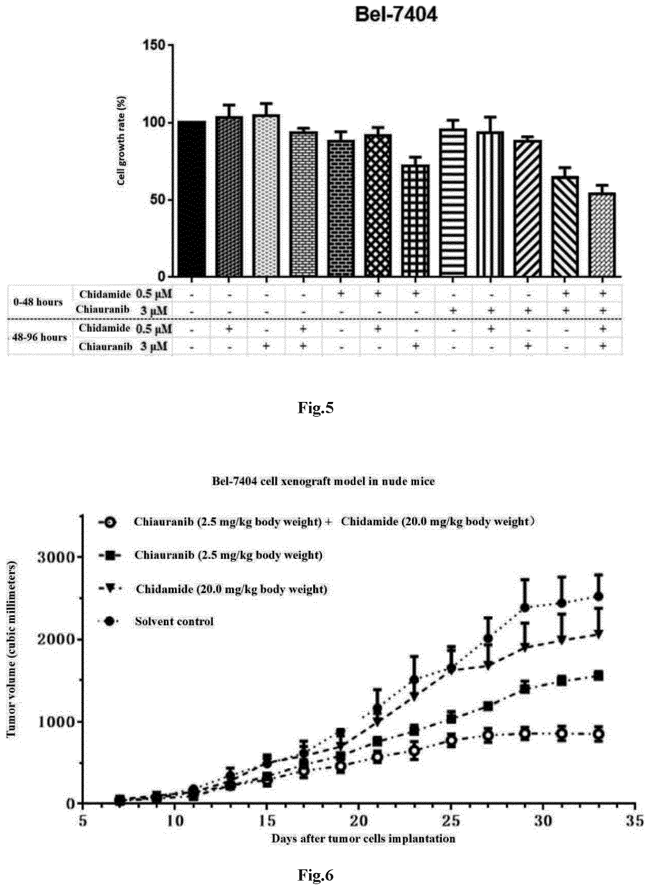

The cells of Bel-7404 at the log phase stage were digested by trypsin, collected, counted, seeded in a 96-well cell culture plate at the density of 5,000 cells/180 $\mu$L in each well, and cultured under 5% $CO_2$ at 37°C. After the cells were cultured overnight, the drugs corresponding to the grouping and the final concentrations from 0 to 48 hours as shown in FIG 5, were added, and the final volume in each well reached 200 $\mu$L. After the cells were cultured for 48 hours, the culture media were aspirated away carefully from the 96-well plate. In each well, 180 $\mu$L of fresh culture media and the drugs corresponding to the grouping and the final concentrations for 48-96 hours as shown in FIG 5, were added (so that the final volume in each well reached 200 $\mu$L). After the cells were cultured for another 48 hours, that is, the total time of the drug treatment reached to 96 hours, the culture media were discarded in the 96-well plate, and the MTS reagents for cell viability assay were added to detect and calculate the cell survival rate in each well.

The experimental result

As shown in FIG 5, in Bel-7404, whether it was at the first 48 hours or at the next 48 hours, the inhibitory effect on cells caused by adding two drugs at the same time was greater than that by adding each of the two drugs separately. It should be noticed that, pretreating the cells with Chidamide monotherapyat the first 48 hours and then treating the cells with Chiauranib monotherapy at the next 48 hours after Chidamide withdrawal, was greatly different from that pretreating the cells with Chiauranib monotherapy at the first 48 hours and then treating the cells with Chidamide monotherapy at the next 48 hours after Chiauranib withdrawal. The pretreatment with Chidamide enabled the cells more sensitive to Chiauranib, but the pretreatment with Chiauranib did not make the cells sensitive to the subsequent effect of Chidamide monotherapy, indicating that the mechanism of the synergistic sensitization resulting from the combination of the two drugs was that the epigenetic modification of the cells by Chidamide changed the sensitivity of the cells to the pharmacological effects of Chiauranib.

Example 6. Experiments of tumor cell xenograft model in nude mice

The experimental method

The cells of Bel-7404 were cultured to expend to a large amount, and maintained at a log phase state. After the number of the cells reached the required amount, they were digested with trypsin, collected, washed twice with a large amount of PBS to remove the trypsin and serum, and centrifuged at 800 rpm for 10 min at room temperature and the cell supernatants were discarded. The cells were resuspended in the FBS-free RPIM-1640 culture medium, and adjusted the cell density to $10^7/300$ $\mu$L.

Under aseptic conditions, each nude mouse was injected subcutaneously on the back with 300 $\mu$L/injection, one injection per nude mouse. A 1 mL disposable medical syringe for injection was used, and the injection site and direction were roughly same for each nude mouse.

One day after cell implanting, the nude mice were randomly divided into four groups (namely, solvent control group, Chiauranib 2.5 mg/kg group, Chidamide 20 mg/kg group and the combined administration group), housed in separate cages after marked, administered in groups and tumor formation of each mouse was observed every day. With the body weight of each nude mouse measured before administration, the nude mice were administered intragastrically at a dosage based on per kilogram of the body weight, that is, 10 μL of CMC-Na solution per gram of body weight for the solvent control group, 10 μL of 0.25 mg/mL Chiauranib-CMC-Na suspension per gram of body weight for the Chiauranib 2.5 mg/kg group, 10 μL of 2 mg/mL Chidamide-CMC-Na suspension per gram of body weight for the Chidamide 20 mg/kg group, and 10 μL of the CMC-Na suspension containing 0.25 mg of Chiauranib and 2 mg of Chidamide per milliliter, per gram of body weight for the combined administration group. Every 2 days, the longest diameter of the tumor (length) and the widest diameter (width) perpendicular to the longest diameter were measured with a vernier caliper. The tumor volume was calculated by the formula TS= length × (width)$^2$/2 and recorded. Each nude mouse was administered intragastrically regularly once a day. The experiment was ended after the last administration on the 33$^{rd}$ day.

The experiment result

As shown in FIG 6, compared with the solvent control group, in the two groups administered the single drug of 2.5 mg/kg Chiauranib or 20 mg/kg Chidamide, the tumor volume of the tumor-bearing nude mice was inhibited to a certain extent, while the final tumor inhibition rate of the combined administration group was higher than the sum of the tumor inhibition rates of the two groups administered the single drug. This demonstrated that the two drugs had synergistically sensitized anti-tumor activity in the tumor-bearing nude mice.

Example 7. Preparation (in 1,000 units) of compound pharmaceutical composition 1 (granules or capsules)

Chidamide solid dispersion formula

| components | amount |
| --- | --- |
| Chidamide | 50 g |
| povidone | 250 g |
| medicinal ethanol | 8,000 mL |

Compound granule formula

| components | amount |
| --- | --- |
| Chidamide solid dispersion | 180 g |
| Chiauranib | 50 g |
| povidone | 10 g |
| microcrystalline cellulose | 160 g |
| lactose | 100 g |
| magnesium stearate | 5 g |
| medicinal ethanol | 75 mL |

Preparation process

(1) Based on the amounts of the components in the Chidamide solid dispersion formula, Chidamide, povidone and medicinal ethanol were weighed, mixed, and stirred until all the components were completely dissolved at 80°C to obtain a transparent solution. The solution was subjected to rotary evaporation or spray drying to prepare a white powder, that is, Chidamide solid dispersion;

(2) Based on the amounts of the components in the compound granule formula, the Chidamide solid dispersion, Chiauranib, povidone, microcrystalline cellulose and lactose were weighed. In a wet granulator, the excipients were added and mixed uniformly, and then medicinal ethanol was added to obtain granules in wet granulation. The obtained granules were dried in an oven or a fluidized bed dryer, and sieved with a 20 mesh screen, to obtain the granules containing pharmaceutical auxiliary substances.

(3) The granules obtained in step (2) were mixed with magnesium stearate in proportions for 2 min to obtain total mixed granules.

(4) The total mixed granules obtained in step (3) were packaged to obtain granules, or filled hollow capsules into obtained capsules.

Example 8. Preparation (in 1,000 units) of compound pharmaceutical composition 2 (granules, capsules or tablets)

Chidamide solid dispersion formula

| components | amount |
|---|---|
| Chidamide | 50 g |
| copovidone | 250 g |
| medicinal ethanol | 5,500 mL |

Compound granule formula

| components | amount |
|---|---|
| Chidamide solid dispersion | 60 g |
| Chiauranib | 90 g |
| crospovidone | 50 g |
| microcrystalline cellulose | 160 g |
| mannitol | 140 g |
| magnesium stearate | 5 g |

Preparation process

(1) Based on the amounts of the components in the Chidamide solid dispersion formula, Chidamide, copovidone and medicinal ethanol were weighed, mixed, and stirred until all the components were completely dissolved at 80°C to obtain a transparent solution. The solution was subjected to rotary evaporation or spray drying to prepare approximately a white powder, that is, Chidamide solid dispersion;

(2) Based on the amounts of the components in the compound granule formula, the Chidamide solid dispersion, Chiauranib, crospovidone, microcrystalline cellulose and mannitol were weighed. The excipients were added to a three-dimensional mixer and mixed uniformly, to prepare granules in dry granulation. The obtained granules were sieved with a 20 mesh screen, to obtain the granules containing pharmaceutical auxiliary substances.

(3) The granules obtained in step (2) were mixed with magnesium stearate in proportions for 2 min to obtain total mixed granules.

(4) The total mixed granules obtained in step (3) were packaged to obtain rapid-release granules, or filled hollow capsules to obtain capsules, or compressed into obtained tablets.

Example 9. Preparation (in 1,000 units) of compound pharmaceutical composition 3 (granules or tablets)

Chidamide solid dispersion formula

| components | amount |
|---|---|
| Chidamide | 60 g |
| hydroxypropyl methyl cellulose | 300 g |
| medicinal ethanol | 6,000 mL |
| water | 1,600 mL |

Compound granule formula

| components | amount |
|---|---|
| Chidamide solid dispersion | 360 g |
| Chiauranib | 30 g |
| crospovidone | 80 g |
| microcrystalline cellulose | 200 g |
| lactose | 190 g |
| magnesium stearate | 8 g |

Preparation process

(1) Based on the amounts of the components in the Chidamide solid dispersion formula, hydroxypropyl methyl cellulose was weighed and dissolved in water in the formula amount, in which Chidamide was added then, stirred until it was dissolved at 80°C to obtain a transparent solution. The solution was subjected to rotary evaporation or spray drying to prepare an off-white powder, that is, Chidamide solid dispersion.

(2) Based on the amounts of the components in the compound granule formula, the Chidamide solid dispersion, Chiauranib, crospovidone, microcrystalline cellulose and lactose were weighed. The excipients were added to a three-dimensional mixer and mixed uniformly, to prepare granules in dry granulation. The obtained granules were sieved with a 20 mesh screen, to obtain the granules containing pharmaceutical auxiliary substances.

(3) The granules obtained in step (2) were mixed with magnesium stearate in proportions for 2 min to obtain total mixed granules.

(4) The total mixed granules obtained in step (3) were packaged to obtain rapid-release granules, or compressed into obtained tablets.

Example 10. Preparation (in 1,000 units) of compound pharmaceutical composition 4 (capsules)

Formula of a drug coating solution for Chidamide

| components | amount |
|---|---|
| Chidamide | 20 g |
| povidone | 100 g |
| medicinal ethanol | 3,000 mL |

Formula of a drug coating solution for Chiauranib

| components | amount |
|---|---|
| Chiauranib | 60 g |
| povidone | 25 g |
| medicinal ethanol | 500 mL |

Pill core formula

| components | amount |
|---|---|
| blank pill core (microcrystalline cellulose) | 150 g |

Preparation process

(1) Based on the amounts of the components in the formula of the drug coating solution for Chidamide, Chidamide, povidone and medicinal ethanol were weighed, mixed, and stirred until all the components were completely dissolved at 80°C, to obtain a transparent solution, that is, a coating solution for Chiauranib.

(2) Based on the amounts of the components in the formula of the drug coating solution for Chiauranib, povidone and medicinal ethanol were weighed. Povidone was dissolved in the ethanol. Chiauranib was dispersed uniformly in the solution of povidone, to obtain a drug coating solution for Chiauranib.

(3) Coating: The blank pill core was accurately weighed, and applied with the drugs by spraying the drug coating solution for Chidamide and the drug coating solution for Chiauranib separately in a fluidized bed; the pill core after spraying was weighed to calculate the rate of drug application, so the combined drug pellets containing Chidamide and Chiauranib were obtained.

(4) The pellets obtained in step (3) by coating were filled hollow capsules into obtained capsules.

Example 11. Preparation of (in 1,000 units) compound pharmaceutical composition 5 (capsules)

Formula of combined drug coating solution for Chidamide and Chiauranib

| components | amount |
|---|---|
| Chidamide | 5 g |
| Chiauranib | 100 g |

(continued)

| components | amount |
| --- | --- |
| copovidone | 30 g |
| medicinal ethanol | 1,000 mL |

Pill core formula:

| components | amount |
| --- | --- |
| blank pill core (microcrystalline cellulose) | 100 g |

Preparation process

(1) Based on the amounts of the components in the formula of the combined drug coating solution for Chidamide and Chiauranib, Chidamide, povidone and medicinal ethanol were weighed, mixed, and stirred until they were completely dissolved at 80°C, to obtain a transparent solution. Chiauranib was dispersed uniformly in the transparent solution of Chidamide, to obtain a combined drug coating solution for Chidamide and Chiauranib.

(2) Coating: The blank pill core was accurately weighed, and applied with the drugs by spraying the combination of Chidamide and Chiauranib coating liquid in a fluidized bed; the pill core after spraying was weighed to calculate the rate of drug application, so the combined drug pellets containing Chidamide and Chiauranib were obtained.

(3) The pellets obtained in step (2) by coating were filled hollow capsules into obtained capsules.

The above-prepared formulations were tested for drug dissolution *in vitro* in a 0.1 mol/L hydrochloric acid medium, according to the Pharmacopoeia of the People's Republic of China 2015 edition. As a result, more than 85% of drugs were dissolved within 15 min, which met the release requirements.

**Claims**

1. A combination of a histone deacetylase inhibitor and a protein kinase inhibitor for use in treating or preventing hepatocellular carcinoma;

   wherein the histone deacetylase inhibitor is Chidamide or a pharmaceutically acceptable salt thereof; and
   the protein kinase inhibitor is Chiauranib or a pharmaceutically acceptable salt thereof.

2. The combination for use according to claim 1, wherein the histone deacetylase inhibitor is Chidamide and the protein kinase inhibitor is Chiauranib.

3. A pharmaceutical composition for use in treating or preventing hepatocellular carcinoma, comprising a histone deacetylase inhibitor and a protein kinase inhibitor as active pharmaceutical ingredients, and optionally a pharmaceutically acceptable excipient and/or carrier;

   wherein the histone deacetylase inhibitor is Chidamide or a pharmaceutically acceptable salt thereof; and
   the protein kinase inhibitor is Chiauranib or a pharmaceutically acceptable salt thereof.

4. The pharmaceutical composition for use according to claim 3, wherein the active pharmaceutical ingredients consist of Chiauranib and Chidamide.

5. The pharmaceutical composition for use according to claim 4, wherein the pharmaceutically acceptable excipient and/or carrier includes povidone, copovidone, hydroxypropyl methyl celluloses and polyvinyl capralactam-polyvinyl acetate-polyethylene glycol graft copolymers.

6. The pharmaceutical composition for use according to claim 4 or 5, wherein in a unit dosage, the amount of Chidamide is 5-100 mg and the amount of Chiauranib is 5-100 mg.

7. The pharmaceutical composition for use according to claim 4 or 5, wherein in a unit dosage, the amount of Chidamide is 5-60 mg and the amount of Chiauranib is 10-100 mg.

8. The pharmaceutical composition for use according to any one of claims 4 to 7, wherein the pharmaceutical composition is in a form of granules, solid dispersions, capsules or tablets.

9. The pharmaceutical composition for use according to claim 8, comprising pharmaceutically acceptable excipients and/or carriers selected from the group consisting of microcrystalline cellulose, povidone, copovidone, lactose, mannitol, crospovidone and sodium carboxymethyl cellulose.

10. A kit for use for treating or preventing hepatocellular carcinoma, comprising Chidamide or a pharmaceutically acceptable salt thereof and Chiauranib or a pharmaceutically acceptable salt thereof as active ingredients, wherein Chidamide or a pharmaceutically acceptable salt thereof and Chiauranib or a pharmaceutically acceptable salt thereof are administered simultaneously or sequentially.

11. The kit for use according to claim 10, comprising Chidamide or a pharmaceutically acceptable salt thereof administered first and Chiauranib or a pharmaceutically acceptable salt thereof administered second as active ingredients.


**Patentansprüche**

1. Kombination aus einem Histon-Deacetylase-Inhibitor und einem Proteinkinase-Inhibitor zur Verwendung bei der Behandlung von oder Vorbeugung vor hepatozellulärem Karzinom;

   wobei der Histon-Deacetylase-Inhibitor Chidamid oder ein pharmazeutisch akzeptables Salz davon ist; und der Proteinkinase-Inhibitor ist Chiauranib oder ein pharmazeutisch akzeptables Salz davon.

2. Kombination zur Verwendung nach Anspruch 1, wobei der Histon-Deacetylase-Inhibitor Chidamid und der Proteinkinase-Inhibitor Chiauranib ist.

3. Pharmazeutische Zusammensetzung zur Verwendung bei der Behandlung von oder Vorbeugung vor hepatozellulärem Karzinom, umfassend einen Histon-Deacetylase-Inhibitor und einen Proteinkinase-Inhibitor als pharmazeutische Wirkstoffe und gegebenenfalls einen pharmazeutisch akzeptablen Hilfsstoff und/oder Träger;

   wobei der Histon-Deacetylase-Inhibitor Chidamid oder ein pharmazeutisch akzeptables Salz davon ist; und der Proteinkinase-Inhibitor ist Chiauranib oder ein pharmazeutisch akzeptables Salz davon.

4. Pharmazeutische Zusammensetzung zur Verwendung nach Anspruch 3, wobei die pharmazeutischen Wirkstoffe aus Chiauranib und Chidamid bestehen.

5. Pharmazeutische Zusammensetzung zur Verwendung nach Anspruch 4, wobei der pharmazeutisch annehmbare Hilfsstoff und/oder Träger Povidon, Copovidon, Hydroxypropylmethylcellulosen und Polyvinylcapralactam-Polyvinylacetat-Polyethylenglykol-Pfropfcopolymere enthält.

6. Pharmazeutische Zusammensetzung zur Verwendung nach Anspruch 4 oder 5, wobei in einer Dosierungseinheit die Menge an Chidamid 5-100 mg und die Menge an Chiauranib 5-100 mg beträgt.

7. Pharmazeutische Zusammensetzung zur Verwendung nach Anspruch 4 oder 5, wobei in einer Dosierungseinheit die Menge an Chidamid 5-60 mg und die Menge an Chiauranib 10-100 mg beträgt.

8. Pharmazeutische Zusammensetzung zur Verwendung nach einem der Ansprüche 4 bis 7, wobei die pharmazeu-

**EP 3 838 273 B1**

tische Zusammensetzung in Form von Granulat, festen Dispersionen, Kapseln oder Tabletten vorliegt.

9. Pharmazeutische Zusammensetzung zur Verwendung nach Anspruch 8, umfassend pharmazeutisch annehmbare Hilfsstoffe und/oder Träger, ausgewählt aus der Gruppe bestehend aus mikrokristalliner Cellulose, Povidon, Copovidon, Lactose, Mannit, Crospovidon und Natriumcarboxymethylcellulose.

10. Kit zur Verwendung zur Behandlung von oder Vorbeugung vor hepatozellulärem Karzinom, umfassend Chidamid oder ein pharmazeutisch annehmbares Salz davon und Chiauranib oder ein pharmazeutisch annehmbares Salz davon als aktive Bestandteile, wobei Chidamid oder ein pharmazeutisch annehmbares Salz davon und Chiauranib oder ein pharmazeutisch annehmbares Salz davon gleichzeitig oder aufeinanderfolgend verabreicht werden.

11. Kit zur Verwendung nach Anspruch 10, umfassend Chidamid oder ein pharmazeutisch annehmbares Salz davon, das als erstes verabreicht wird, und Chiauranib oder ein pharmazeutisch annehmbares Salz davon, das als zweites verabreicht wird, als aktive Bestandteile.

**Revendications**

1. Combinaison d'un inhibiteur d'histone désacétylase et d'un inhibiteur de protéine kinase pour le traitement ou la prévention du carcinome hépatocellulaire ;

   dans laquelle l'inhibiteur de l'histone désacétylase est le Chidamide ou un de ses sels pharmaceutiquement acceptables ; et
   l'inhibiteur de protéine kinase est le Chiauranib ou un de ses sels pharmaceutiquement acceptables.

2. Combinaison pour une utilisation selon la revendication 1, dans laquelle l'inhibiteur de l'histone désacétylase est le Chidamide et l'inhibiteur de la protéine kinase est le Chiauranib.

3. Composition pharmaceutique pour une utilisation dans le traitement ou la prévention d'un carcinome hépatocellulaire, comprenant un inhibiteur d'histone désacétylase et un inhibiteur de protéine kinase en tant qu'ingrédients pharmaceutiques actifs, et éventuellement un excipient et/ou un support pharmaceutiquement acceptable ;

   dans laquelle l'inhibiteur de l'histone désacétylase est le Chidamide ou un de ses sels pharmaceutiquement acceptables ; et
   l'inhibiteur de protéine kinase est le Chiauranib ou un de ses sels pharmaceutiquement acceptables.

4. Composition pharmaceutique pour utilisation selon la revendication 3, dans laquelle les principes pharmaceutiques actifs sont le Chiauranib et le Chidamide.

5. Composition pharmaceutique pour une utilisation selon la revendication 4, dans laquelle l'excipient et/ou le support pharmaceutiquement acceptable comprend de la povidone, de la copovidone, des hydroxypropylméthylcelluloses et des copolymères greffés de capralactame de polyvinyle, d'acétate de polyvinyle et de polyéthylène glycol.

6. Composition pharmaceutique pour une utilisation selon la revendication 4 ou 5, dans laquelle, dans un dosage unitaire, la quantité de Chidamide est de 5 à 100 mg et la quantité de Chiauranib est de 5 à 100 mg.

7. Composition pharmaceutique pour une utilisation selon la revendication 4 ou 5, dans laquelle, dans un dosage unitaire, la quantité de Chidamide est de 5 à 60 mg et la quantité de Chiauranib est de 10 à 100 mg.

8. Composition pharmaceutique pour une utilisation selon l'une quelconque des revendications 4 à 7, dans laquelle la composition pharmaceutique se présente sous la forme de granulés, de dispersions solides, de gélules ou de comprimés.

9. Composition pharmaceutique selon la revendication 8, comprenant des excipients et/ou des supports pharmaceutiquement acceptables choisis dans le groupe constitué par la cellulose microcristalline, la povidone, la copovidone, le lactose, le mannitol, la crospovidone et la carboxyméthylcellulose sodique.

10. Kit à utiliser pour traiter ou prévenir un carcinome hépatocellulaire, comprenant le Chidamide ou un de ses sels pharmaceutiquement acceptables et le Chiauranib ou un de ses sels pharmaceutiquement acceptables comme ingrédients actifs, dans lequel le Chidamide ou un de ses sels pharmaceutiquement acceptables et le Chiauranib ou un de ses sels pharmaceutiquement acceptables sont administrés simultanément ou séquentiellement.

11. Kit à utiliser selon la revendication 10, comprenant le Chidamide ou un sel pharmaceutiquement acceptable de celui-ci administré en premier lieu et le Chiauranib ou un sel pharmaceutiquement acceptable de celui-ci administré en second lieu en tant que principes actifs.

Bel-7402

a

Bel-7404

b

Fig.1

## SMMC-7721

a

## Bel-7404

b

Fig.2

Fig.3

a

b

Fig.4

## Bel-7404

| | | | | | | | | | | | | | | |
|---|---|---|---|---|---|---|---|---|---|---|---|---|---|---|
| 0-48 hours | Chidamide 0.5 μM | - | - | - | - | + | + | + | - | - | - | + | + |
| | Chiauranib 3 μM | - | - | - | - | - | - | - | + | + | + | + | + |
| 48-96 hours | Chidamide 0.5 μM | - | + | - | + | - | + | - | - | + | - | - | + |
| | Chiauranib 3 μM | - | - | + | + | - | - | + | - | - | + | - | + |

Fig.5

### Bel-7404 cell xenograft model in nude mice

- ○ Chiauranib (2.5 mg/kg body weight) + Chidamide (20.0 mg/kg body weight)
- ■ Chiauranib (2.5 mg/kg body weight)
- ▼ Chidamide (20.0 mg/kg body weight)
- ● Solvent control

Fig.6

## REFERENCES CITED IN THE DESCRIPTION

**Patent documents cited in the description**

- CN 201810943005

**Non-patent literature cited in the description**

- *J Hepatol,* 2005, vol. 42, 210-217
- **YINDONG KANG et al.** Studies on the Immunomodulation Effect of Histone Deacetylase Inhibitors through Dendritic Cells. *Chinese Journal of Transplantation,* 2009, vol. 3 (2
- **ZHOU Y. et al.** *Cancer Science,* 2017, vol. 108 (3), 469-477
- **MANMAN D. et al.** *Experimental Cell Research,* 2018, vol. 369 (2), 356-362
- **HARDIE ; HANKS.** The Protein Kinase Facts Book. Academic Press, 1995, vol. I,II
- **HANKS ; HUNTER.** *FASEB,* 1995, vol. 9, 576-596
- **KNIGHTON.** *Science,* 1991, vol. 253, 407-414
- **HILES.** *Cell,* 1992, vol. 70, 419-429
- **KUNZ.** *Cell,* 1993, vol. 73, 585-596
- **GARCIA-BUSTOS.** *EMBO J.,* 1994, vol. 13, 2352-2361
- **HEINRICH ; GRIFFITH.** *Blood,* 2000, vol. 96 (3), 925-32
- **GAESTEL et al.** *Current Medicinal Chemistry,* 2007, vol. 14, 2214-2234
- Pharmacopoeia of the People's Republic of China. 2015